# EUROPÄISCHE PATENTSCHRIFT

(11) **EP 0 716 743 B1**
(45) Veröffentlichungstag und Bekanntmachung des Hinweises auf die Patenterteilung: **14.06.2000**
(21) Anmeldenummer: 94927502.8
(22) Anmeldetag: 25.08.1994
(51) Int. Cl.: G01N 33/28, G01N 31/22, C10L 1/00

(54) **VERFAHREN ZUM NACHWEIS VON NAPHTHYLAMINEN IN MINERALÖLEN**
METHOD OF DETECTING NAPHTHYLAMINES IN MINERAL OILS
PROCEDE PERMETTANT DE DETECTER LA PRESENCE DE NAPHTHYLAMINES DANS DES HUILES MINERALES

(30) Priorität: 04.09.1993 DE 4329953
(43) Veröffentlichungstag der Anmeldung: 19.06.1996
(73) Patentinhaber: BASF AKTIENGESELLSCHAFT, 67056 Ludwigshafen (DE)
(72) Erfinder: DYLLICK-BRENZINGER, Rainer, D-69469 Weinheim (DE); SCHLÖSSER, Ulrike, D-67071 Ludwigshafen (DE); KURTZ, Walter, D-67098 Bad Dürkheim (DE); LAMM, Gunther, D-67454 Hassloch (DE)
(86) Internationale Anmeldenummer: EP9402824
(87) Internationale Veröffentlichungsnummer: WO9507460

(56) Entgegenhaltungen:
- EP-A- 0 147 704
- EP-A- 0 149 125
- US-A- 4 209 302
- US-A- 5 229 298
- ANAL. CHEM. (1982), 54(1), 91-6 CODEN: ANCHAM;ISSN: 0003-2700, 1982 Tomkins, B. A. et al 'Determination of polycyclic aromatic amines in natural and synthetic crudes'

## Beschreibung

Die vorliegende Erfindung betrifft ein neues Verfahren zum Nachweis von Naphthylaminen, die als Markierungsmittel in Mineralölen enthalten sind, wobei man das Naphthylamin durch Behandeln des Mineralöls mit einem wäßrigen Medium extrahiert und in der wäßrigen Phase mit einem Diazoniumsalz eines aromatischen Amins unter Bildung eines Azofarbstoffs kuppelt.

Ein weiterer Gegenstand der vorliegenden Erfindung betrifft die Verwendung von speziellen Naphthylaminen als Markierungsmittel für Mineralöle sowie Mineralöle, enthaltend diese Naphthylamine.

Aus der US-A-4 209 302 ist die Verwendung von 1- oder 2-(3-Morpholinopropylamino)naphthalin, 1- oder 2-(3-Dimethylaminopropylamino)naphthalin oder 1- oder 2-(3-Diethylamninopropylamino)naphthalin als Markierungsmittel bekannt.

Der Nachweis dieser Verbindungen wird dabei durch Umsetzung der entsprechenden Naphthylamine mit diazotiertem 2-Chlor-4-nitroanilin vorgenommen. Es hat sich jedoch gezeigt, daß diese Markierungs- und Nachweismethode noch nicht voll befriedigend ist, da u.a. die Stabilität des Diazoniumsalzes gering ist.

Aus Anal. Chem., Band 54, Seiten 91 bis 96, 1982 ist bekannt, daß Mineralöle u.a. 1- oder 2-Aminonaphthaline enthalten können.

Aufgabe der vorliegenden Erfindung war es nun, ein neues Verfahren zum Nachweis von mit Naphthylaminen markierten Mineralölen bereitzustellen. Das neue Verfahren sollte in einfacher Weise durchführbar sein. Dabei sollten selbst noch sehr kleine Mengen an Markierstoff durch eine starke Farbreaktion sichtbar gemacht werden können. Schließlich sollte der Marker nicht durch einfache Extraktion mit Wasser aus dem markiertem Mineralöl entfernbar sein.

Es wurde nun gefunden, daß der Nachweis der Anwesenheit von Naphthylaminen der Formel I 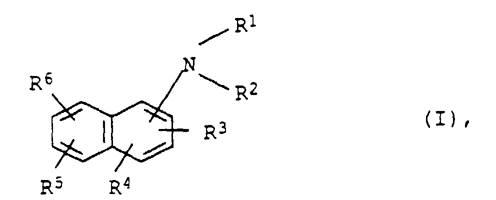 in der
- R¹ und R²: unabhängig voneinander jeweils Wasserstoff, C₁-C₁₈-Alkyl, das gegebenenfalls substituiert ist und durch 1 bis 3 Sauerstoffatome in Etherfunktion oder 1 bis 3 C₁-C₄-Alkyliminogruppen unterbrochen sein kann, C₅-C₇-Cycloalkyl, C₃-C₁₈-Alkenyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, und
- R³,R⁴,R⁵ und R⁶: unabhängig voneinander jeweils Wasserstoff, C₁-C₈-Alkyl, Benzyl, Cyano, Nitro, C₁-C₄-Alkanoyl, C₁-C₄-Alkanoylamino, Benzoylamino, Hydroxysulfonyl oder einen Rest der Formel OL¹, COOL¹, NL¹L² oder CONL¹L², worin L¹ und L² jeweils für Wasserstoff, gegebenenfalls substituiertes C₁-C₁₈-Alkyl, C₅-C₇-Cycloalkyl oder gegebenenfalls substituiertes Phenyl stehen, oder R³ zusammen mit R² C₃-Alkylen, das gegebenenfalls durch Hydroxy substituiert ist, bedeuten, wobei, wenn die Reste NR¹R² und NL¹L² peri-ständig sind, R¹ und L¹ zusammen auch Isopropyliden bedeuten können,
in Mineralölen vorteilhaft gelingt, wenn man das Naphthylamin der Formel I durch Behandeln des Mineralöls mit einem wäßrigen Medium extrahiert und in der wäßrigen Phase, gegebenenfalls in Gegenwart eines Puffers, mit einem Diazoniumsalz, das sich von einem Amin aus der Aminoanthrachinonreihe ableitet, unter Bildung eines Azofarbstoffs kuppelt.

Alle in den Naphthylaminen auftretenden Alkyl- und Alkenylgruppen können sowohl geradkettig als auch verzweigt sein.

Im erfindungsgemäßen Sinn sind unter Alkenylresten im wesentlichen solche Reste zu verstehen, die 1 bis 3 Doppelbindungen aufweisen.

Wenn in der obengenannten Formel I substituierte Alkylgruppen auftreten, so können, sofern nicht anders vermerkt, als Substituenten z.B. Hydroxy, Amino, Morpholinyl oder Phenyl, in Betracht kommen. Die Alkylgruppen weisen dabei in der Regel 1 oder 2 Substituenten auf.

Wenn in der obengenannten Formel I substituierte Phenylgruppen auftreten, so können als Substituenten z.B. C₁-C₁₂-Alkyl, Hydroxy oder C₁-C₄-Alkoxy in Betracht kommen. Die Phenylgruppen weisen dabei in der Regel 1 bis 3 Substituenten auf.

Wenn R¹ und R² zusammen mit dem verbindenden Stickstoffatom einen 5- oder 6-gliedrigen gesättigten heterocyclischen Rest, der ein weiteres Heteroatom aufweisen kann, bedeuten, so können dafür z.B. Pyrrolidinyl, Piperidinyl, Morpholinyl, Piperazinyl oder N-(C₁-C₄-Alkyl)piperazinyl in Betracht kommen.

Reste R¹, R², R³, R⁴, R⁵, R⁶, L¹, und L² sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl, 2-Ethylhexyl oder Isooctyl.

Reste R¹, R², L¹ und L² sind weiterhin z.B., 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 5-Hydroxy-3-oxapentyl, Benzyl, 1-Phenylethyl, 2-Phenylethyl, 2-Aminoethyl, 2- oder 3-Aminopropyl, 2- oder 4-Aminobutyl, 2-Morpholinylethyl, 2- oder 3-Morpholinylpropyl, 2- oder 4-Morpholinylbutyl, Cyclopentyl, Cyclohexyl, Cycloheptyl, Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl oder 2,4-Dimethoxyphenyl.

Reste R¹ und R² sind weiterhin z.B. Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 2-Dimethylaminoethyl, 2-Diethylaminoethyl, 2- oder 3-Dimethylaminopropyl, 2- oder 3-Diethylaminopropyl, 2- oder 4-Dimethylaminopropyl, 2- oder 4-Diethylaminobutyl, 3,6-Dimethyl-3,6-diazaheptyl, 3,6,9-Trimethyl-3,6,9-triazadecyl, 2-(1-Methoxyethoxy)ethyl, 2-(1-Ethoxyethoxy)ethyl, 2-(1-Isobutoxyethoxy)ethyl, 2- oder 3-(1-Methoxyethoxy)propyl, 2- oder 3-(1-Ethoxyethoxy)propyl, 2- oder 3-(1-Isobutoxyethoxy)propyl, Allyl, Prop-1-en-1-yl, Methallyl, Ethallyl, Pentenyl, Pentadienyl, Hexadienyl, 3,7-Dimethylocta-1,6-dien-1-yl, Undec-10-en-1-yl, 6,10-Dimethylundeca-5,9-dien-2-yl, 3,7,11-Trimethyldodeca-1,6,10-trien-1-yl, 3,7,11-Trimethyldodeca-2,6, 10-trien-1-yl, Octadec-9-en-1-yl, Octadeca-9,12-dien-1-yl, Octadeca-9,12,15-trien-1-yl oder 6,10,14-Trimethylpentadeca-5,9, 13-trien-2-yl,

Reste R³, R⁴, R⁵ und R⁶ sind weiterhin z.B. Formyl, Acetyl, Propionyl, Butyryl, Isobutyryl, Formylamino, Acetylamino, Propionylamino, Butyrylamino, Isobutyrylamino, Hydroxy, Methoxy, Ethoxy, Propoxy, Isopropoxy, Butoxy, Isobutoxy, sec-Butoxy, tert-Butoxy, Pentyloxy, Isopentyloxy, Neopentyloxy, tert-Pentyloxy, Hexyloxy, Heptyloxy, Octyloxy, Isooctyloxy, 2-Ethylhexyloxy, Amino, Mono- oder Dimethylamino, Mono- oder Diethylamino, Mono- oder Dipropylamino, Mono- oder Diisopropylamino, Mono- oder Dibutylamino, N-Methyl-N-ethylamino, Mono- oder Dicyclohexylamino, N-Methyl-N-cyclohexylamino, Phenylamino, N-Phenyl-N-methylamino, Carbamoyl, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, N-Methyl-N-ethylcarbamoyl, Phenylcarbamoyl, Methoxycarbonyl, Ethoxycarbonyl, Propoxycarbonyl, Isopropoxycarbonyl, Butoxycarbonyl oder Phenoxycarbonyl.

Reste R² und R³ zusammen sind z.B. 1,3-Propylen oder 2-Hydroxy-1,3-propylen.

Bevorzugt ist ein Verfahren zum Nachweis von Naphthylamninen der Formel I, in der
- R¹ und R²: unabhängig voneinander jeweils Wasserstoff, C₁-C₁₅-Alkyl, das gegebenenfalls substituiert ist und durch 1 bis 3 Sauerstoffatome in Etherfunktion unterbrochen sein kann, Cyclohexyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, und
- zwei der Reste R³, R⁴, R⁵ und R⁶: jeweils Wasserstoff und die anderen beiden unabhängig voneinander jeweils Wasserstoff, C₁-C₄-Alkyl, C₁-C₄-Alkoxy, Hydroxy, Nitro, C₁-C₄-Alkanoylamino, Benzoylamino, einen Rest der Formel NL¹L², worin L¹ und L² unabhängig voneinander jeweils für Wasserstoff oder C₁-C₄-Alkyl stehen, Cyano oder Carboxyl bedeuten, wobei, wenn die Reste NR¹R² und NL¹L² peri-ständig sind, R¹ und L¹ zusammen auch Isopropyliden bedeuten können.

Besonders bevorzugt ist ein Verfahren zum Nachweis von Naphthylaminen der Formel I, in der R¹ Wasserstoff, R² C₁-C₁₅-Alkyl, das durch 1 bis 3 Sauerstoffatome in Etherfunktion unterbrochen sein kann, oder Cyclohexyl und einer der Reste R³, R⁴, R⁵ und R⁶ Hydroxy oder C₁-C₄-Alkoxy und die anderen Wasserstoff bedeuten.

Bevorzugt ist auch ein Verfahren zum Nachweis von Naphthylaminen der Formel I, wobei man das Naphthylamin der Formel I durch Behandeln des Mineralöls mit einer, vorzugsweise sauren, wäßrigen Lösung eines Diazoniumsalzes, das sich von einem Amin aus der Aminoanthrachinonreihe ableitet, extrahiert und anschließend, gegebenenfalls in Gegenwart eines Puffers, mit dem Diazoniumsalz unter Bildung eines Azofarbstoffs kuppelt.

Als Amin aus der Anthrachinonreihe ist 1-Aminoanthrachinon besonders hervorzuheben.

Unter Mineralölen im erfindungsgemäßen Sinn sind beispielsweise Treibstoffe, wie Benzin, Kerosin oder Dieselöl, oder Öle, wie Heizöl oder Motorenöl, zu verstehen.

Zum Markieren von Mineralöl werden die Naphthylamine der Formel I entweder in Substanz oder in Form von Lösungen angewandt. Als Lösungsmittel eignen sich vorzugsweise aromatische Kohlenwasserstoffe, wie Dodecylbenzol, Diisopropylnaphthalin oder ein Gemisch höherer Aromaten, das unter dem Namen Shellsol® AB (Fa. Shell) handelsüblich ist. Um eine hohe Viskosität der resultierenden Lösungen zu vermeiden, wählt man im allgemeinen eine Konzentration an Naphthylamin I von 30 bis 50 Gew.-%, bezogen auf die Lösung.

Mittels des erfindungsgemäßen Verfahrens gelingt es sehr einfach, die Naphthylamine der Formel I in Mineralölen nachzuweisen, selbst wenn die Markierungssubstanzen nur in einer Konzentration von ungefähr 10 ppm oder darunter vorliegen.

Wenn man das Naphthylamin der Formel I mit einer sauren wäßrigen Lösung eines Diazoniumsalzes extrahiert, so sind geeignete Säuren z.B. anorganische oder organische Säuren, wie Salzsäure, Schwefelsäure, Salpetersäure, Phosphorsäure, Ameisensäure, Essigsäure oder Propionsäure. Die sauren wäßrigen Lösungen weisen im allgemeinen eine Säurekonzentration von 0,5 bis 20 Gew.-% auf.

Geeignete wäßrige Medien zur Extraktion der Naphthylamine der Formel I aus dem Mineralöl sind z.B. Wasser oder Gemische von Wasser mit Säuren und/oder wassermischbaren organischen Lösungsmitteln und/oder anorganischen Substanzen.

Als Säuren kommen dabei die obengenannten Säuren in der angegebenen Konzentration in Betracht.

Wassermischbare organische Lösungsmittel sind z.B. Alkohole, wie Methanol, Ethanol, Propanol, Isopropanol, Ethylenglykol oder 1,2-Propylenglykol, Ether, wie 2-Methoxyethanol, 2-Ethoxyethanol, 2-Butoxyethanol, 2-(2-Methoxyethoxy)ethanol, 1-Methoxypropan-2-ol oder Tetrahydrofuran, Carbonsäureamide, wie N,N-Dimethylformamid oder N-Methylpyrrolidon, Propylencarbonat, Dimethylsulfoxid oder Sulfolan. Der Anteil an wassermischbarem organischem Lösungsmittel liegt im allgemeinen bei 1 bis 50 Gew.-%, bezogen auf das Gewicht des wäßrigen Mediums.

Anorganische Substanzen sind z.B. Salze, wie Alkalihalogenide, Aluminiumhalogenide, Zinkhalogenide oder Ammoniumsalze.

Die Anwendung von wäßriger Säure, die gegebenenfalls noch ein wassermischbares organisches Lösungsmittel enthält, als wäßriges Medium ist bevorzugt.

Um zu einer optimalen Kupplungsreaktion, die gegebenenfalls in Gegenwart eines Lösungsmittels verläuft, und daraus resultierend zu einer Optimierung der Ausbeute an Azofarbstoff zu gelangen, empfiehlt es sich den pH-Wert durch Anwendung von Puffersubstanzen zu kontrollieren und die Reaktionspartner im günstigen Molverhältnis (Naphthylamin : Diazoniumsalz 1 : 500 bis 1 : 10, vorzugsweise 1 : 100 bis 1 : 20) anzuwenden.

Geeignete Puffersubstanzen sind z.B. Alkaliacetate, Monoalkalicitrate, Alkalidihydrogenphosphate, wobei insbesondere jeweils die Natriumsalze zu nennen sind, oder solche Puffersysteme, wie sie im Handbook of Chemistry and Physics, 65th Ed., 1984-1985, Seiten D148 bis D150, genannt sind.

Geeignete Anionen, die als Gegenionen zu den Diazoniumkationen in Betracht kommen, sind die dort üblichen Anionen, wie Chlorid, Hydrogensulfat, Sulfat, Dihydrogenphosphat, Monohydrogenphosphat, Phosphat, Tetrafluoroborat, Tetrachlorozinkat oder Acetat.

In manchen Fällen ist es vorteilhaft, noch geringe Mengen an Alkalisalzen von Arylsulfonsäuren, z.B. das Natriumsalz der Naphthalin-1,5-disulfonsäure, als Stabilisator für die Diazoniumsalze zuzusetzen.

Zur Nachweisreaktion verwendet man in der Regel eine wäßrige Lösung eines Diazoniumsalzes, die 0,1 bis 2 Gew.-%, bezogen auf das Gewicht der Lösung, an Diazoniumsalz aufweist. Je Gewichtsteil markiertes Mineralöl werden dabei im allgemeinen 0,01 bis 1 Gew.-Teile Diazoniumsalzlösung angewandt.

Der Nachweis der Naphthylamine der Formel I in Mineralölen gelingt weiterhin vorteilhaft, wenn man das Naphthylamin der Formel I durch Behandeln des Mineralöls mit einem wäßrigen Medium extrahiert und in der wäßrigen Phase, gegebenenfalls in Gegenwart eines Puffers, mit einem Diazoniumsalz, das sich von einem Amin aus der Aminoanthrachinonreihe ableitet und sich in festem Aggregatzustand auf einem Träger befindet, unter Bildung eines Azofarbstoffs kuppelt.

Geeignete Träger sind anorganische Materialien, wie Aktivkohle, Molekularsiebe, Kieselgur, Titandioxid, Aluminiumoxid oder Calciumchlorid, oder organische Materialien, wie Cellulosefasern, Baumwolle, Holzschliff, Polystyrol oder Polyvinylchlorid.

Nach der Zugabe des auf dem Träger befindlichen Diazoniumsalzes zum wäßrigen Extrakt des Naphthylamins der Formel I wird das Diazoniumsalz ganz oder teilweise gelöst und so für die Azokupplung zur Verfügung gestellt.

Geeignet als Träger ist auch ein Papierstreifen, z.B. aus Filterpapier. Er kann mit einer Lösung eines der obengenannten Diazoniumsalze imprägniert und getrocknet werden. (Durch Lagerung der imprägnierten Papierstreifen im Trockenen und im Dunkeln kann dabei die Zersetzung des Diazoniumsalzes verhindert werden).

Wenn man einen solchen imprägnierten Papierstreifen in den wäßrigen Extrakt eintaucht, kommt es an seiner Oberfläche durch die Bildung eines Azofarbstoffs zu einer Farbreaktion. Auf diesem Weg können die Naphthylamine der Formel I auf äußerst einfache Weise nachgewiesen werden.

Eine vorteilhafte Methode besteht auch darin, zum wäßrigen Extrakt einige Schnipsel des imuprägnierten Papierstreifens zu geben und gegebenenfalls kurzzeitig zu erwärmen.

Als vorteilhaft hat sich der Nachweis der Naphthylamine der Formel I mittels eines "Diazopapiers" oder einer "Diazofolie" erwiesen. Diese Diazoindikatoren werden durch Tränken eines Papiers, z. B. eines Filterpapiers, oder einer Dünnschichtchromatographiefolie, die z. B. mit Cellulose beschichtet ist, mit der entsprechenden Diazoniumsalzlösung hergestellt, wobei man den Bereich unbehandelt läßt, der beim Entwickeln direkt in Kontakt mit dem wäßrigen Extrakt kommt. Durch Eintauchen eines so hergestellten Papier- oder Folienstreifens in den wäßrigen Extrakt läßt sich die Azofarbstoffausbeute nach Art einer Papierchromatographie optimieren. Der dabei sich bildende Azofarbstoff bleibt am Start und wird durch laufend nachgeliefertes Naphthylamin aufkonzentriert und ist deshalb gut nachzuweisen.

Bei den Naphthylaminen der Formel I handelt es sich um übliche an sich bekannte Produkte aus der Farbstoffherstellung. Sie sind leicht zugänglich.

Die vorliegende Erfindung betrifft weiterhin die Verwendung von Naphthylaminen der Formel Ia 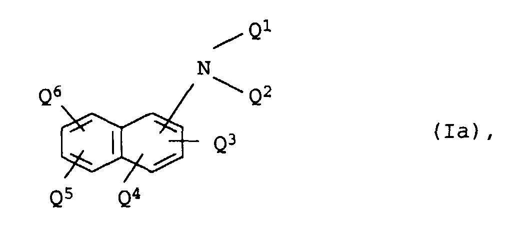 in der
Q¹ Wasserstoff, Q² C₁-C₁₅-Alkyl, das durch 1 bis 3 Sauerstoffatome in Etherfunktion unterbrochen sein kann, oder Cyclohexyl, und einer der Reste Q³, Q⁴, Q⁵ und Q⁶ Hydroxy oder C₁-C₄-Alkoxy und die anderen Wasserstoff bedeuten,
zum Markieren von Mineralölen.

Bezüglich der beispielhaften Aufzählung der Reste Q2 bis Q⁶ sei auf die voranstehend vorgenommenen Ausführungen, betreffend die Reste R¹ bis R⁶ hingewiesen.

Unter Markierung im erfindungsgemäßen Sinn ist ein Zusatz der Naphthylamine der Formel Ia in solcher Konzentration zu verstehen, daß die Mineralöle dadurch für das menschliche Auge überhaupt nicht verändert sind, wobei jedoch die Naphthylamine der Formel Ia durch die hier näher beschriebenen Nachweismethoden leicht und deutlich sichtbar detektierbar sind.

Die Naphthylamine der Formel Ia eignen sich insbesondere zum Markieren von Mineralölen, bei denen eine Kennzeichnung gefordert wird, z.B. aus steuerlichen Gründen. Um die Kosten der Kennzeichnung gering zu halten, strebt man dabei an, für die Markierung möglichst geringe Mengen an Markierungsmittel anzuwenden.

Die Markierung der Mineralöle mit den Naphthylaminen der Formel Ia kann z.B. nach der Methode erfolgen, wie sie oben für die Naphthylamine der Formel I bereits ausführlich beschrieben ist.

Der Nachweis der als Markierungastoffe angewandten Naphthylamine der Formel Ia in Mineralölen gelingt vorteilhaft, wenn man das Naphthylamin der Formel Ia durch Behandeln des Mineralöls mit einem wäßrigen Medium extrahiert und in der wäßrigen Phase, gegebenenfalls in Gegenwart eines Puffers, mit einem Diazoniumsalz, das sich von einem Amin aus der Aminoanthrachinon-, Aminonaphthalin-, Anilin-, Aminothiophen-, Aminothiazol- oder Aminobenzisothiazolreihe ableitet, unter Bildung eines Azofarbstoffs kuppelt.

Der Nachweis der als Markierungsstoffe angewandten Naphthylamine der Formel Ia in Mineralölen gelingt besonders vorteilhaft, wenn man das Naphthylamin der Formel Ia durch Behandeln des Mineralöls mit einer, vorzugsweise sauren, wäßrigen Lösung eines Diazoniumsalzes, das sich von einem Amin aus der Aminoanthrachinon-, Aminonaphthalin-, Anilin-, Aminothiophen-, Aminothiazol- oder Aminobenzisothiazolreihe ableitet, extrahiert und anschließend, gegebenenfalls in Gegenwart eines Puffers, mit dem Diazoniumsalz unter Bildung eines Azotarbstoffs kuppelt.

Wie oben bereits näher ausgeführt, ist auch hier eine Verfahrensweise möglich, bei der man Diazoniumsalze, die sich in festem Aggregatzustand auf einem Träger befinden, anwendet.

Als Amin aus der Anthrachinonreihe ist 1-Aminoanthrachinon besonders hervorzuheben.

Geeignete Aminonaphthaline gehorchen z.B. der Formel II 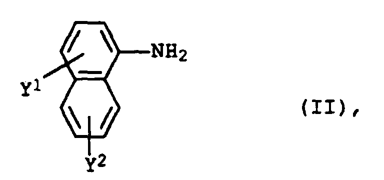 in der Y¹ und Y² unabhängig von einander jeweils Wasserstoff, Hydroxy oder Hydroxysulfonyl bedeuten.

Geeignete Aniline gehorchen z.B. der Formel III 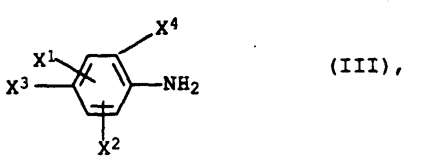 in der
- X¹: Wasserstoff, Halogen, C₁-C₄-Alkoxy, Nitro oder Hydroxysulfonyl,
- X²: Wasserstoff, Halogen, C₁-C₄-Alkoxy, Cyano, Nitro, oder gegebenenfalls durch Methyl, Ethyl, Methoxy oder Ethoxy substituiertes Phenylazo und
- X³: Wasserstoff, C₁-C₄-Dialkylamino, Pyrrolidinyl, Piperidinyl, Morpholinyl, Nitro oder Hydroxysulfonyl und
- X⁴: Wasserstoff, Halogen, Cyano oder einen heterocyclischen Rest, z.B. 3-Phenyl-1,2,4-oxadiazol-5-yl, bedeuten.

Geeignete Aminothiophene, Aminorhiazole oder Aminobenzisothiazole gehorchen z.B. der Formel

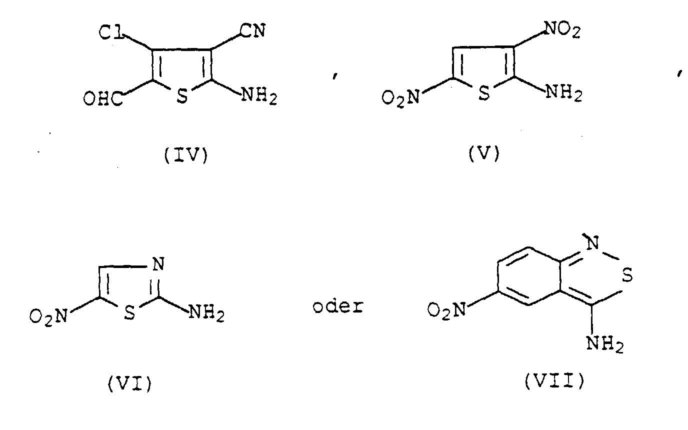

Bezüglich der einzelnen Parameter für die Nachweisreaktionen sei auf die oben für die Naphthylamine der Formel I bereits dargelegten Ausführungen hingewiesen.

Die Naphthylamine der Formel Ia können in den Mineralölen natürlich auch noch mittels an sich bekannten physikalischen Analysemethoden nachgewiesen werden, beispielsweise mittels Gaschromatographie, Hochdruckflüssigkeitschromatographie, Dünnschichtchromatographie oder Säulenchromarographie.

Ein weiterer Gegenstand der vorliegenden Erfindung sind Mineralöle, enthaltend eines oder mehrere Naphthylamine der Formel Ia, wobei 1-Aminonaphthalin und 2-Aminonaphthalin ausgenommen sind.

Die folgenden Beispiele sollen die Erfindung näher erläutern.
a) Herstellung der Reagenzlösungen
   Reagenzlösung A
      Der feuchte Preßkuchen des schwefelsauren Diazoniumsalzes Von 1-Aminoanthrachinon (Feststoffgehalt: 73 Gew.-%) wurde 2,5 gew.-%ig in Wasser gelöst. Der pH-Wert der Lösung betrug 1,2.
   Reagenzlösung B
      Durch wäßrige Diazotierung von 2-(3-Phenyl-1,2,4-oxadiazol-5-yl)anilin mit Natriumnitrit erhielt man eine 0,11-molare wäßrige Diazoniumchloridlösung. Der pH-Wert der Lösung betrug ca. 1,1.
b) Allgemeine Nachweisreaktion in saurer Lösung
   Das jeweilige Naphthylamin wurde in einer Konzentration von 50 ppm in Dieselkraftstoff gelöst und weiter mit Shellsol® AB (Fa. Shell) auf eine Konzentration von 5 ppm verdünnt. 0,9 Volumenteile dieser Lösung wurden mit 0,1 Volumenteilen Reagenzlösung 1 min intensiv geschüttelt. Dann wurde die untere wäßrige Phase visuell beurteilt. Alternativ kann die wäßrige Phase auch noch mit 0,1 bis 0,3 Volumenteilen 50 gew.-%iger Essigsäure versetzt werden. Diese Maßnahme stellt sicher, daß der entstehende Farbstoff vollständig gelöst wird und seine volle Farbstärke zur Geltung kommt.
c) Allgemeine Nachweisreaktion in gepufferter wäßriger Lösung
   Es werden 10 g des mit 10 ppm markierten Dieselkraftstoffs mit 1 g 9 gew.-%iger Salzsäure extrahiert, die wäßrige Phase mit Natronlauge neutralisiert und mit jeweils der vierfachen Menge einer der drei im folgenden genannten Pufferlösungen 1, 2 und 3 versetzt.
   Pufferlösung 1: 5 gew.-%ige wäßrige Citronensäurelösung mit 10 Gew.-%iger Natronlauge auf einen pH-Wert von pH 3,3 eingestellt.
   Pufferlösung 2: 5 gew.-%ige wäßrige Kaliumdihydrogenphosphatlösung mit einem pH-Wert von 4,4.
   Pufferlösung 3: 5 gew.-%ige wäßrige Natriumacetattrihydratlösung mit einem pH-Wert von 8,2.
   Anschließend gibt man 0,1 g der Reagenzlösung A zu.

Die Azokupplungen verlaufen meist recht schnell, jedoch können sie durch kurzzeitiges Erwärmen in den meisten Fällen weiter beschleunigt werden.

Die Ergebnisse der Untersuchungen sind in den folgenden Tabellen aufgeführt. Die Intensität der Färbung wird entweder durch Noten (von 1 bis 5) beurteilt (1: keine Färbung, 5: sehr intensive Färbung) oder es wird die Extinktion angegeben. Außerdem wird angegeben, ob die Farbreaktion sofort eintritt. Schließlich ist auch der Farbton der resultierenden Azoverbindung oder in einigen Fällen ihr Absorptionsmaximum aufgeführt.

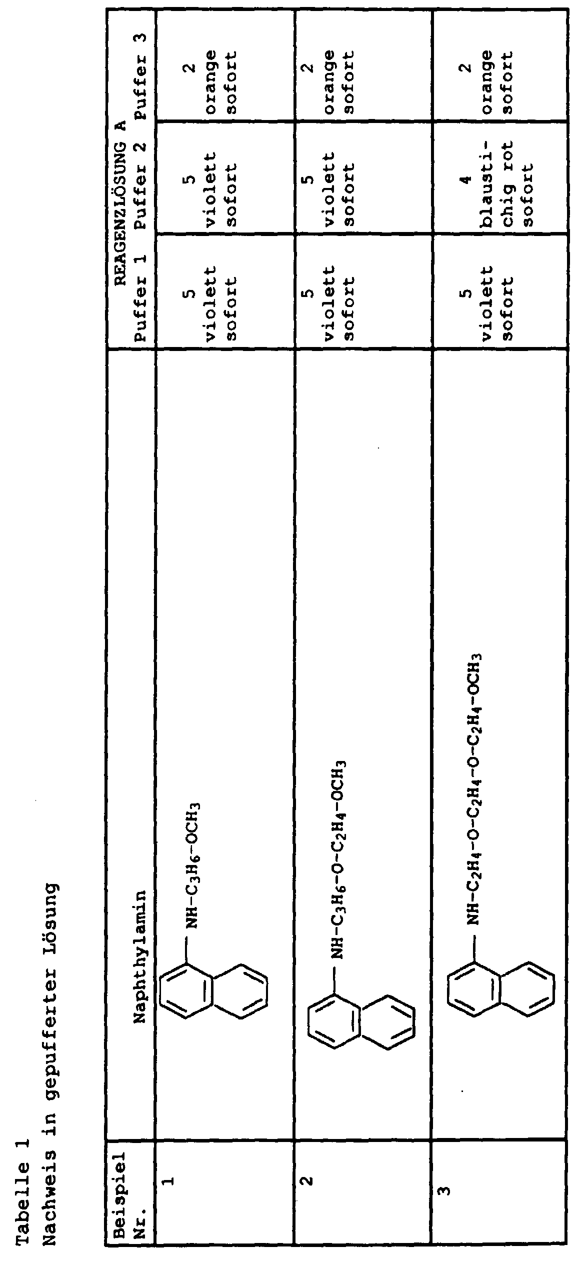

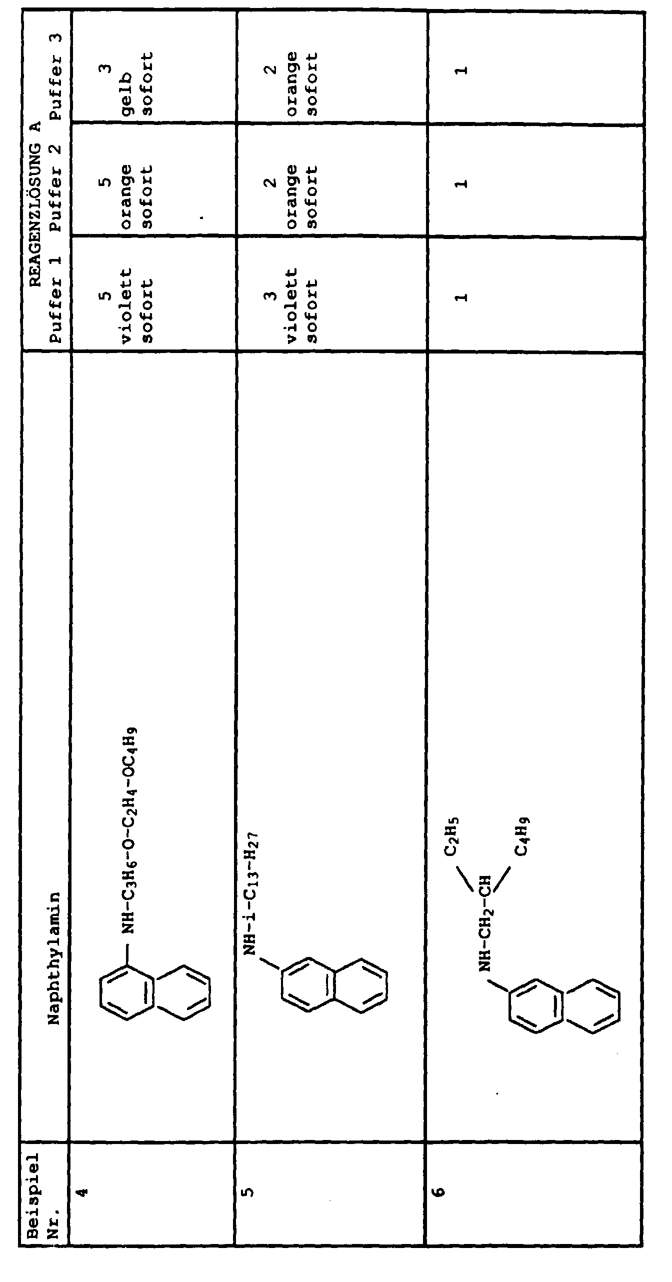

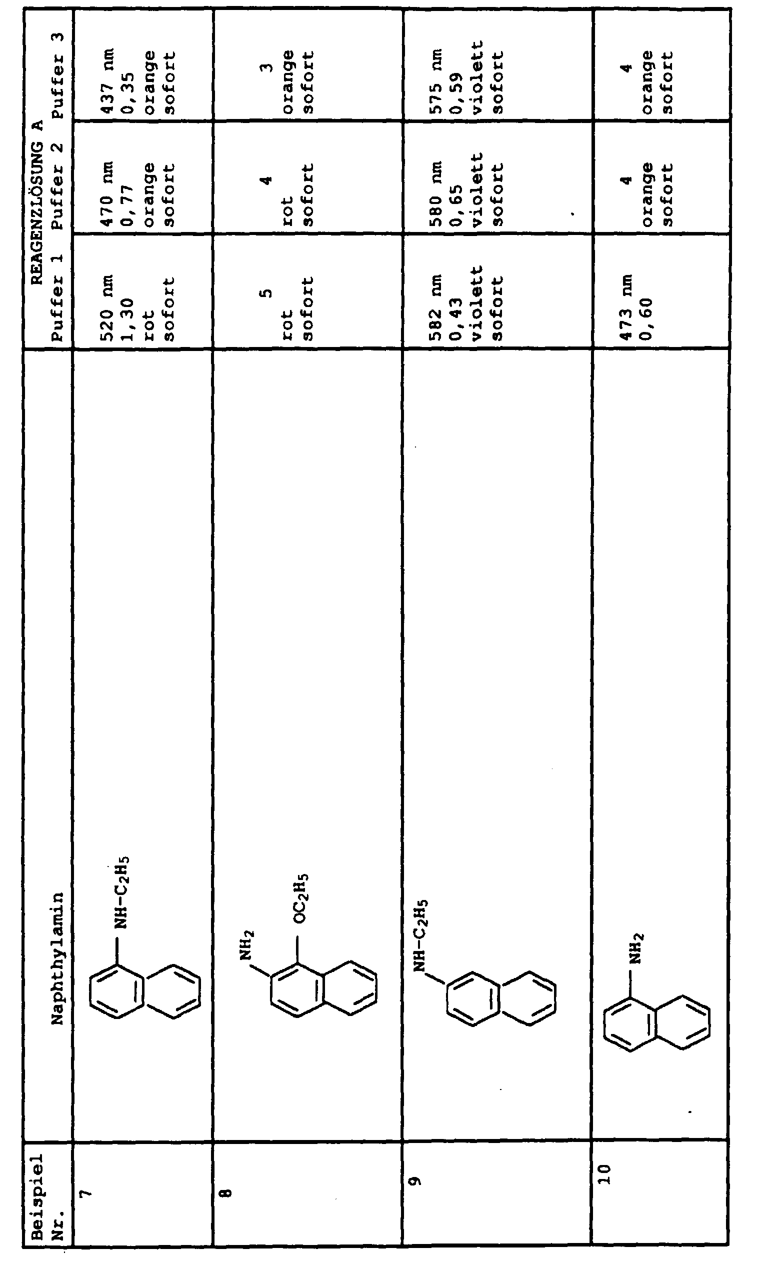

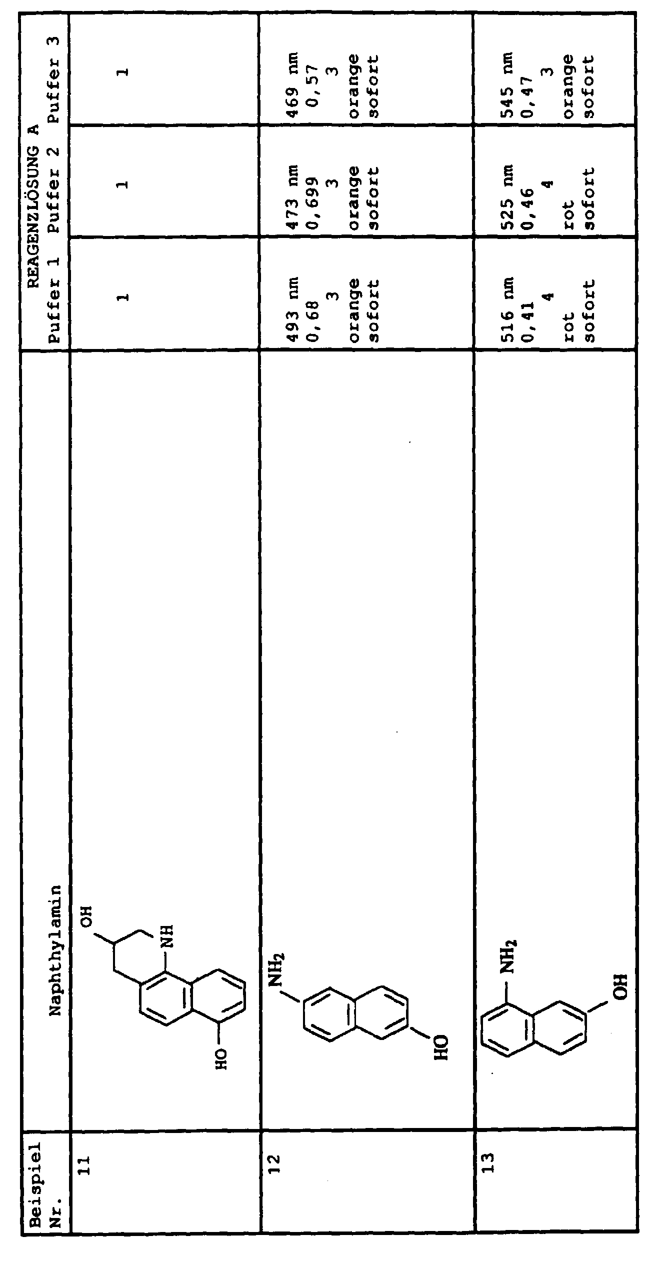

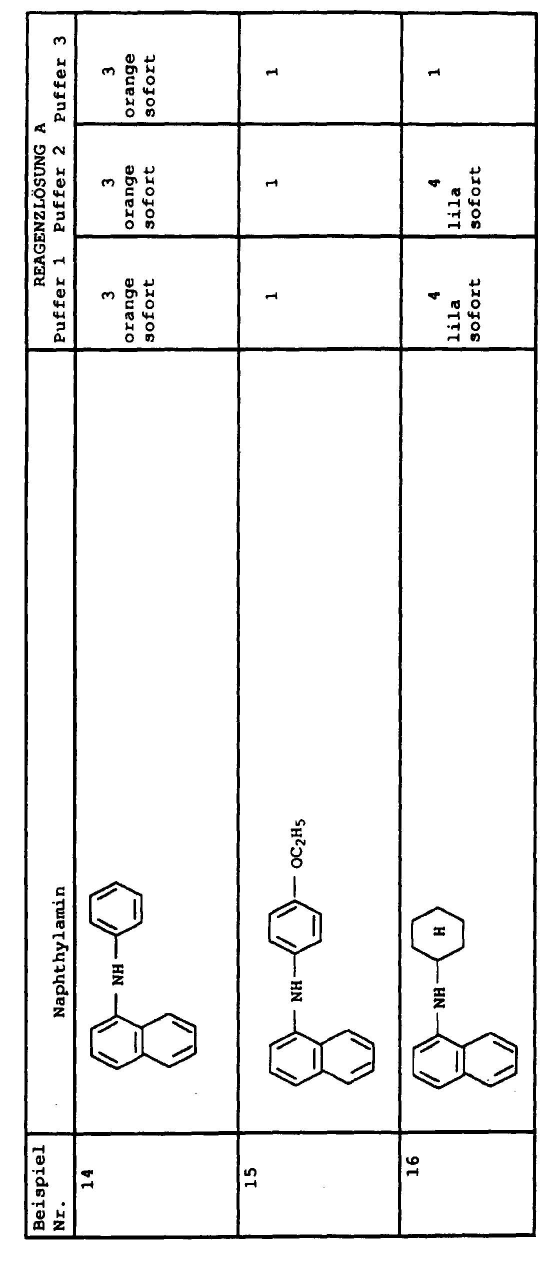

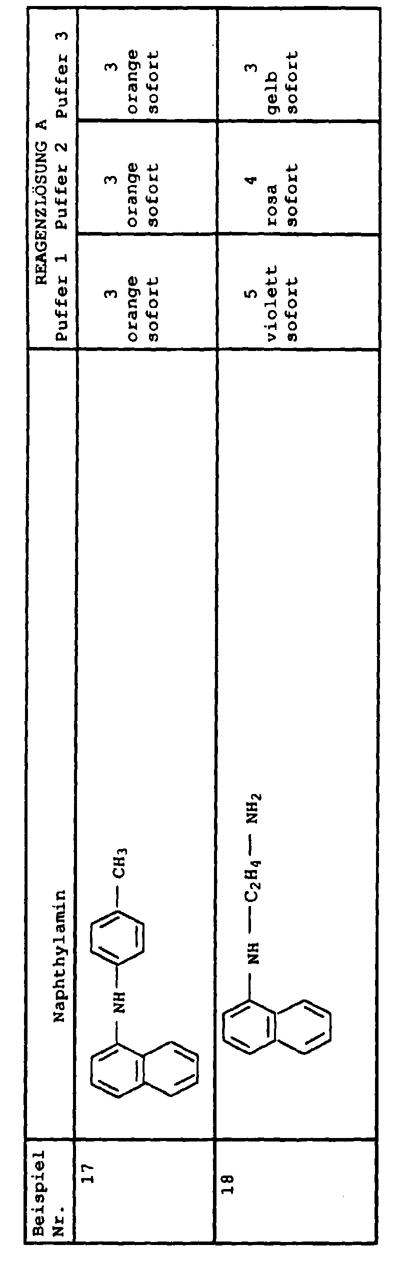

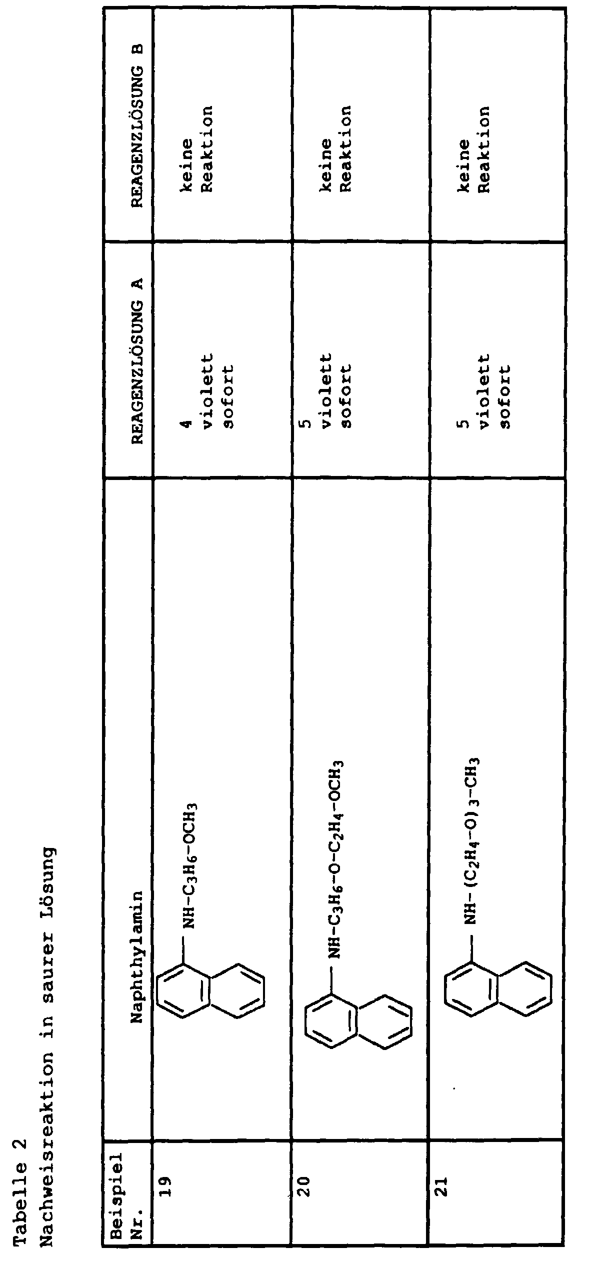

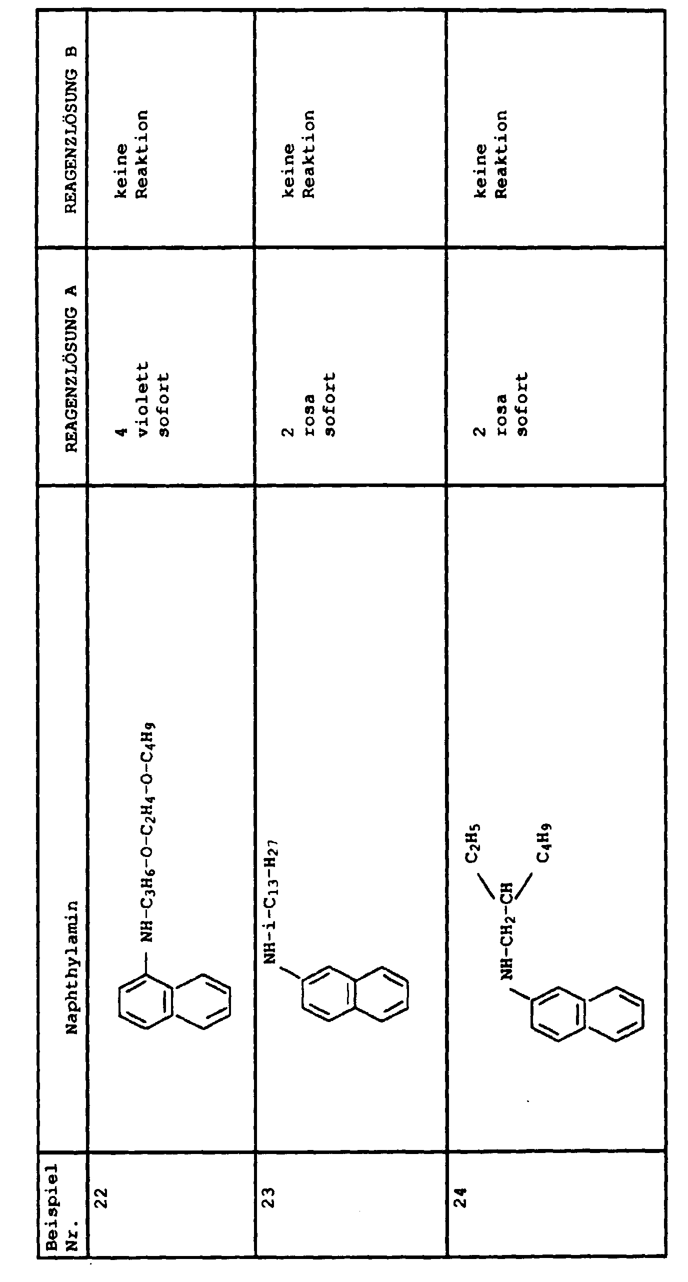

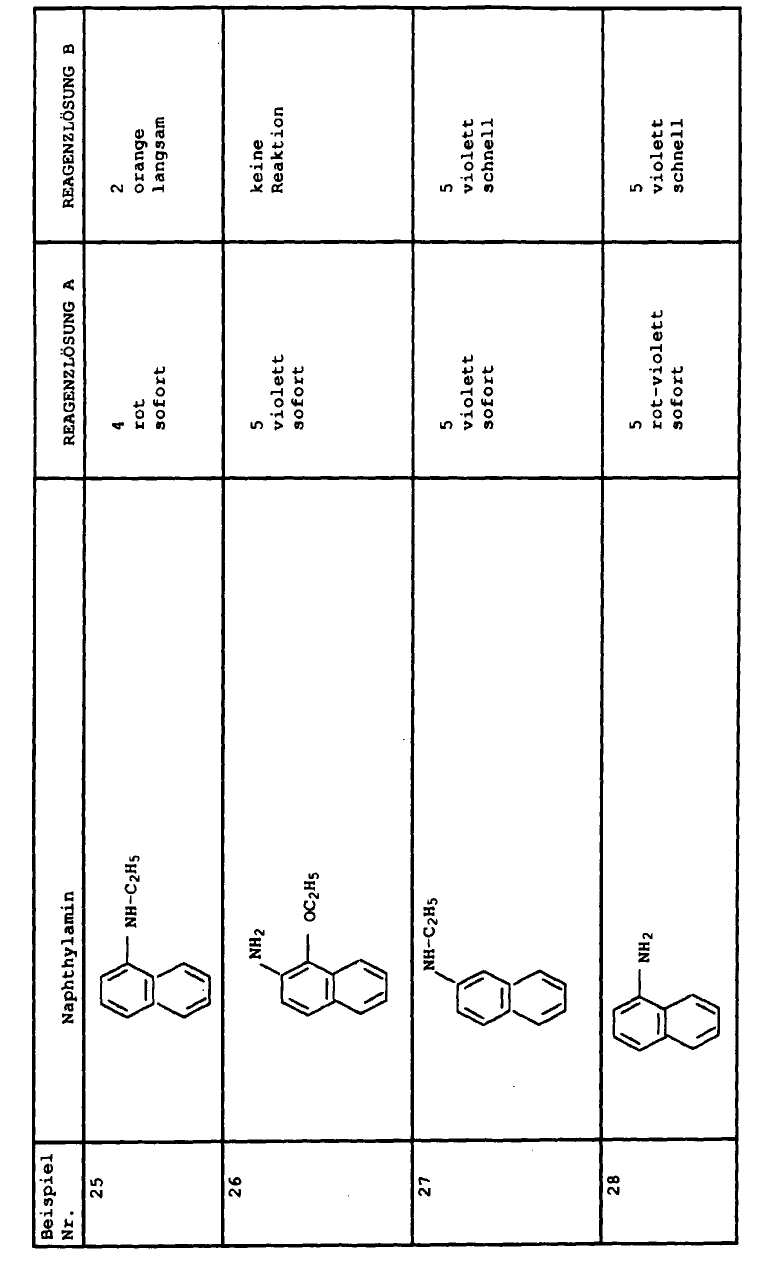

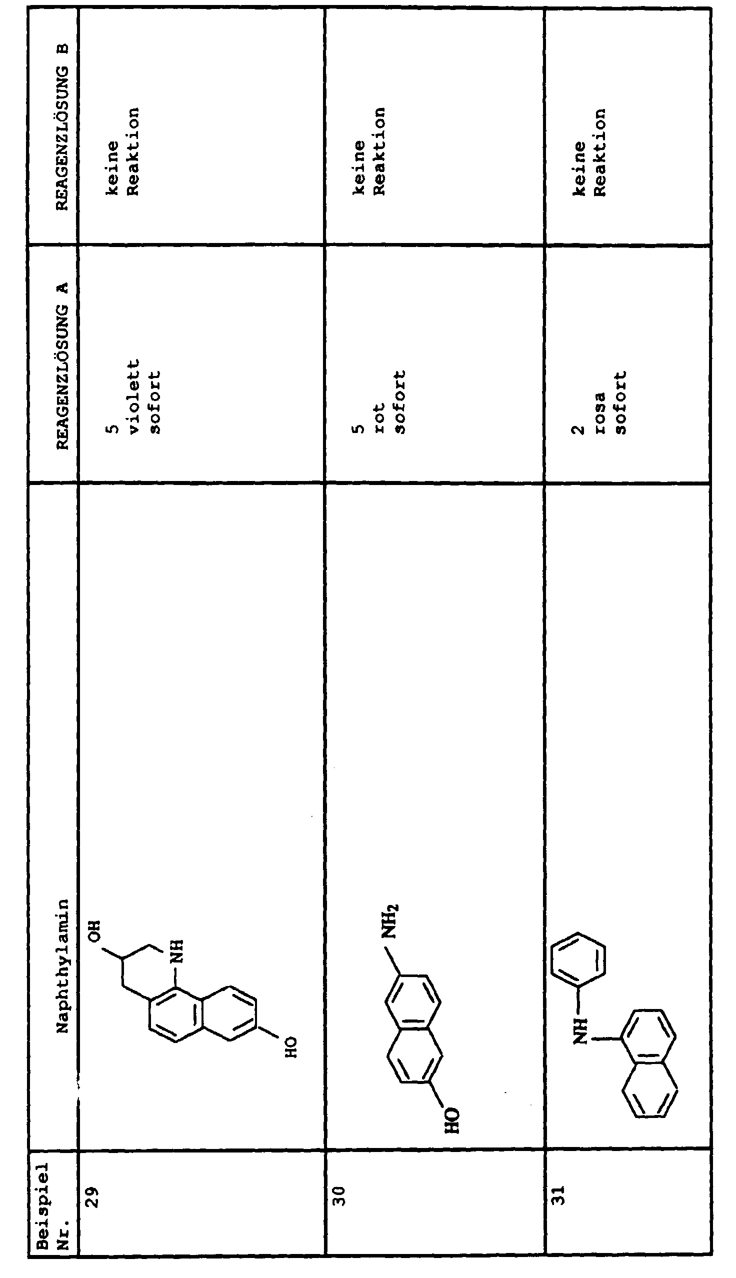

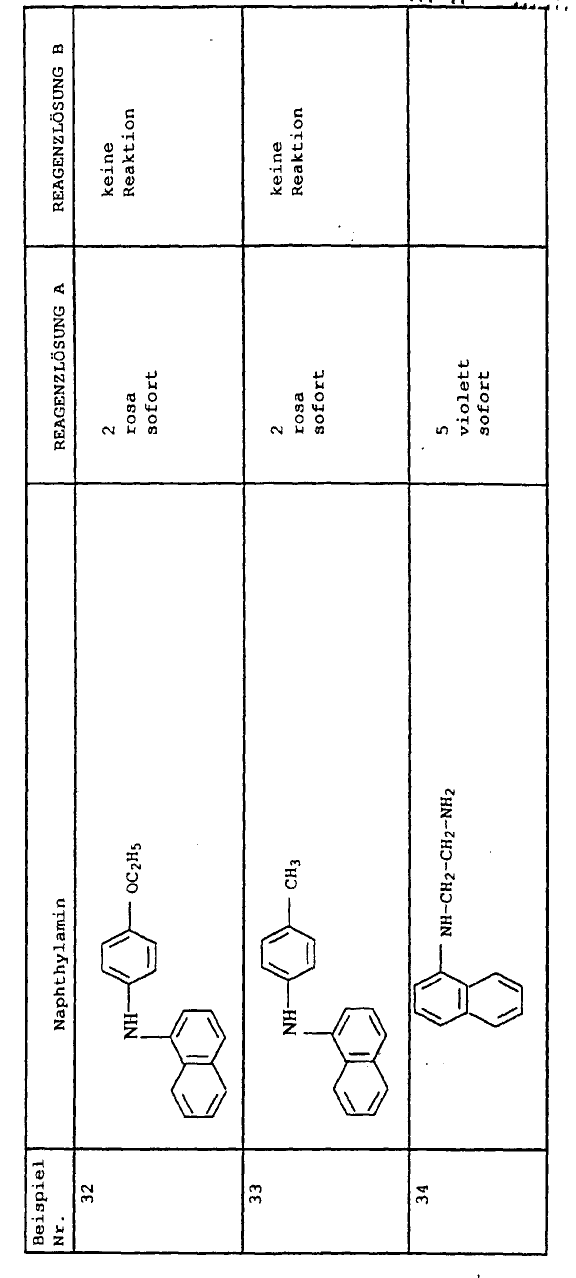

### Beispiel 35

1,5 ml eines mit 10 ppm Tolylperisäure der Formel 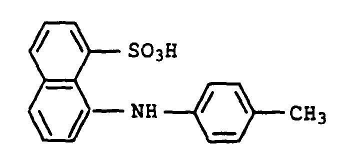 markierten Dieselöls wurden in einem Reaktionsgefäß mit 3 Tropfen einer Lösung von 100 ppm Dinatriumhydrogenphosphat in vollentsalztem Wasser vermischt. Diese Mischung wurde 20 sec intensiv geschüttelt. Anschließend ließ man die Phasen trennen. Die obere ölige Phase war gelb und trüb, die untere eine Spur gelb. Zu diesem zweiphasigen Gemisch gab man nun 2 Tropfen einer 1 gew.-%igen wäßrigen Lösung des schwefelsauren Diazoniumsalzes von 1-Aminoanthrachinon vorsichtig zu.

Wichtig ist, daß die Diazoniumsalzlösung so zugegeben wird, daß sich insgesamt drei Phasen ausbilden. Das Reaktionsgefäß sollte nach der Zugabe der Diazoniumsalzlösung auch nicht mehr geschüttelt werden. An der Phasengrenzfläche zwischen Diazoniumsalz- und Phosphatpufferlösung bildet sich dann ein deutlich sichtbarer, schwarzer Ring. Wird der gleiche Versuch mit einem nicht markierten Heizöl durchgeführt, bleibt die wäßrige Phase ungefärbt und ein schwarzer Ring ist nicht zu sehen.

## Patentansprüche

1. Verfahren zum Nachweis der Anwesenheit von Naphthylaminen der Formel I 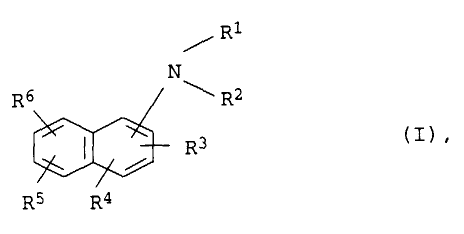 in der
R¹ und R² unabhängig voneinander jeweils Wasserstoff, C₁-C₁₈-Alkyl, das gegebenenfalls substituiert ist und durch 1 bis 3 Sauerstoffatome in Etherfunktion oder 1 bis 3 C₁-C₄-Alkyliminogruppen unterbrochen sein kann, C₅-C₇-Cycloalkyl, C₃-C₁₈-Alkenyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, und
R³,R⁴,R⁵ und R⁶ unabhängig voneinander jeweils Wasserstoff, C₁-C₈-Alkyl, Benzyl, Cyano, Nitro, C₁-C₄-Alkanoyl, C₁-C₄-Alkanoylamino, Benzoylamino, Hydroxysulfonyl oder einen Rest der Formel OL¹, COOL¹, NL¹L² oder CONL¹L², worin L¹ und L² jeweils für Wasserstoff, gegebenenfalls substituiertes C₁-C₈-Alkyl, C₅-C₇-Cycloalkyl oder gegebenenfalls substituiertes Phenyl stehen, oder R³ zusammen mit R² C₃-Alkylen, das gegebenenfalls durch Hydroxy substituiert ist, bedeuten, wobei, wenn die Reste NR¹R² und NL¹L² peri-ständig sind, R¹ und L¹ zusammen auch Isopropyliden bedeuten können,
in Mineralölen, dadurch gekennzeichnet, daß man das Naphthylamin der Formel I durch Behandeln des Mineralöls mit einem wäßrigen Medium extrahiert und in der wäßrigen Phase, gegebenenfalls in Gegenwart eines Puffers, mit einem Diazoniumsalz, das sich von einem Amin aus der Aminoanthrachinonreihe ableitet, unter Bildung eines Azofarbstoffs kuppelt.

2. Verfahren nach Anspruch 1, dadurch gekennzeichnet, daß
R¹ und R² unabhängig voneinander jeweils Wasserstoff, C₁-C₁₅-Alkyl, das gegebenenfalls substituiert ist und durch 1 bis 3 Sauerstoffatome in Etherfunktion unterbrochen sein kann, Cyclohexyl, gegebenenfalls substituiertes Phenyl oder R¹ und R² zusammen mit dem sie verbindenden Stickstoffatom einen 5- oder 6-gliedrigen gesättigten heterocyclischen Rest, der ein weiteres Heteroatom aufweisen kann, und
zwei der Reste R³, R⁴,R⁵ und R⁶ jeweils Wasserstoff und die anderen beiden unabhängig voneinander jeweils Wasserstoff, C₁-C₄-Alkyl, C₁-C₄-Alkoxy, Hydroxy, Nitro, C₁-C₄-Alkanoylamino, Benzoylamino, einen Rest der Formel NL¹L², worin L¹ und L² unabhängig voneinander für Wasserstoff oder C₁-C₄-Alkyl stehen, Cyano oder Carboxyl bedeuten, wobei, wenn die Reste NR¹R² und NL¹L² peri-ständig sind, R¹ und L¹ zusammen auch Isopropyliden bedeuten können.

3. Verfahren nach Anspruch 1, dadurch gekennzeichnet, daß R¹ Wasserstoff, R² C₁-C₁₅-Alkyl, das durch 1 bis 3 Sauerstoffatome in Etherfunktion unterbrochen sein kann, oder Cyclohexyl und einer der Reste R³, R⁴,R⁵ und R⁶ Hydroxy oder C₁-C₄-Alkoxy und die anderen Wasserstoff bedeuten.

4. Verfahren nach Anspruch 1, dadurch gekennzeichnet, daß man das Naphthylamin der Formel I durch Behandeln des Mineralöls mit einer wäßrigen Lösung eines Diazoniumsalzes, das sich von einem Amin aus der Aminoanthrachinonreihe ableitet, extrahiert und anschließend, gegebenenfalls in Gegenwart eines Puffers, mit dem Diazoniumsalz unter Bildung eines Azofarbstoffs kuppelt.

5. Verwendung von Naphthylaminen der Formel Ia 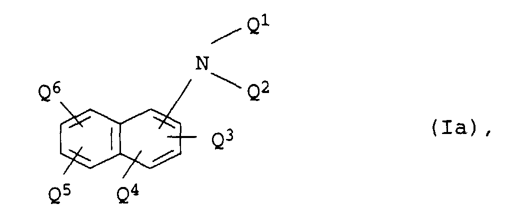 in der
Q¹ Wasserstoff, Q² C₁-C₁₅-Alkyl, das durch 1 bis 3 Sauerstoffatome in Etherfunktion unterbrochen sein kann, oder Cyclohexyl, und einer der Reste Q³, Q⁴, Q⁵ und Q⁶ Hydroxy oder C₁-C₄-Alkoxy und die anderen Wasserstoff bedeuten,
zum Markieren von Mineralölen.

6. Mineralöl, enthaltend eines oder mehrere der Naphthylamine der Formel Ia gemäß Anspruch 5.

## Claims

1. A method for detecting the presence of naphthylamines of the formula I 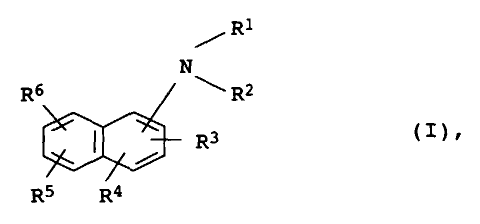 where
R¹ and R² are each, independently of one another, hydrogen, C₁-C₁₈-alkyl which is unsubstituted or substituted and can be interrupted by 1 to 3 oxygen atoms in ether functionalities or 1 to 3 C₁-C₄-alkylimino groups, or C₅-C₇-cycloalkyl, C₃-C₁₈-alkenyl or unsubstituted or substituted phenyl, or R¹ and R² form, together with the nitrogen atom connecting them, a 5- or 6-membered saturated heterocyclic radical which may have another hetero atom, and
R³,R⁴,R⁵ and R⁶, are each, independently of one another, hydrogen, C₁-C₈-alkyl, benzyl, cyano, nitro, C₁-C₄-alkanoyl, C₁-C₄-alkanoylamino, benzoylamino, hydroxysulfonyl or a radical of the formula OL¹, COOL¹, NL¹L² or CONL¹L², where L¹ and L² are each hydrogen, unsubstituted or substituted C₁-C₁₈-alkyl, C₅-C₇-cycloalkyl or unsubstituted or substituted phenyl, or R³ together with R² is C₃-alkylene which is unsubstituted or substituted by hydroxyl, and, when the radicals NR¹R² and NL¹L² are in the pen positions, R¹ and L¹ can together also be isopropylidene
in mineral oils, which comprises extracting the naphthylamine of the formula I by treating the mineral oil with an aqueous medium and coupling it in the aqueous phase, in the presence or absence of a buffer, with a diazonium salt derived from an amine from the aminoanthraquinone series to form an azo dye.

2. A method as claimed in claim 1, wherein
R¹ and R² are each, independently of one another, hydrogen, C₁-C₁₅-alkyl which is unsubstituted or substituted and can be interrupted by 1 to 3 oxygen atoms in ether functionalities, cyclohexyl, unsubstituted or substituted phenyl, or R¹ and R² form, together with the nitrogen atom connecting them, a 5- or 6-membered saturated heterocyclic radical which may have another hetero atom, and
two of the R³, R⁴, R⁵ and R⁶ radicals are each hydrogen and the other two are each, independently of one another, hydrogen, C₁-C₄-alkyl, C₁-C₄-alkoxy, hydroxyl, nitro, C₁-C₄-alkanoylamino, benzoylamino, a radical of the formula NL¹L² where L¹ and L², independently of one another, are hydrogen or C₁-C₄-alkyl, or cyano or carboxyl, and, when the radicals NR¹R² and NL¹L² are in the peri positions, R¹ and L¹ can together also be isopropylidene.

3. A method as claimed in claim 1, wherein R¹ is hydrogen, R² is C₁-C₁₅-alkyl which can be interrupted by 1 to 3 oxygen atoms in ether functionalities, or cyclohexyl and one of the R³, R⁴, R⁵ and R⁶ radicals is hydroxyl or C₁-C₄-alkoxy and the others are hydrogen.

4. A method as claimed in claim 1, wherein the naphthylamine of the formula I is extracted by treating the mineral oil with an aqueous solution of a diazonium salt derived from an amine from the aminoanthraquinone series, and subsequently, in the presence or absence of a buffer, coupled with the diazonium salt to form an azo dye

5. The use of naphthylamines of the formula Ia 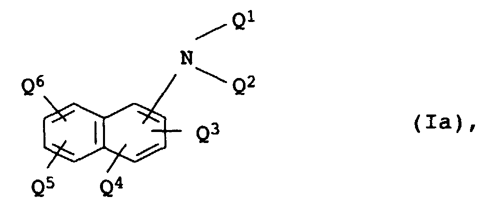 where
Q¹ is hydrogen, Q² is C₁-C₁₅-alkyl which can be interrupted by 1 to 3 oxygen atoms in ether functionalities, or cyclohexyl, and one of the Q³, Q⁴, Q⁵ and Q⁶ radicals is hydroxyl or C₁-C₄-alkoxy and the others are hydrogen,
as tracers in mineral oils.

6. A mineral oil containing one or more napthylamines of the formula Ia as claimed in claim 5.

## Revendications

1. Procédé de détection de présence de naphtylamines de formule I 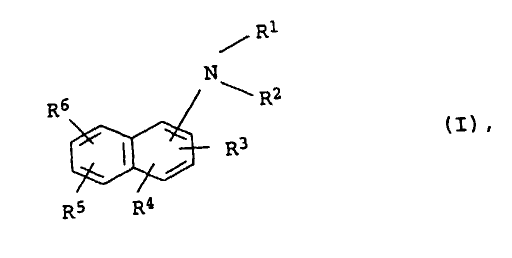 dans laquelle
R¹ et R² représentent chacun indépendamment l'un de l'autre, un atome d'hydrogène, un groupement alkyle en C₁-C₁₈, éventuellement substitué et pouvant être interrompu par 1 à 3 atomes d'oxygène en fonction éther ou par 1 à 3 groupements alkylimino en C₁-C₄, un groupement cycloalkyle en C₅-C₇, alcényle en C₃-C₁₈, un groupement phényle éventuellement substitué ou bien R¹ et R² représentent ensemble avec l'atome d'azote qui les relie, un reste hétérocyclique saturé à 5 ou 6 maillons qui peut présenter un autre hétéroatome, et
R³, R⁴, R⁵ et R⁶ représentent chacun indépendamment les uns des autres, un atome d'hydrogène, un groupement alkyle en C₁-C₈, benzyle, cyano, nitro, alcanoyle en C₁-C₄, alcanoylamino en C₁-C₄, benzoylamino, hydroxysulfonyle ou un reste de formule OL¹, COOL¹, NL¹L² ou CONL¹L², où L¹ et L² sont mis chacun pour un atome d'hydrogène, un groupement alkyle en C₁-C₈ éventuellement substitué, un groupement cycloalkyle en C₅-C₇ ou un groupement phényle éventuellement substitué, ou bien R³ et R² peuvent représenter ensemble un groupement alkylène en C₃, pouvant éventuellement être substitué par un groupement hydroxy, où lorsque les restes NR¹R² et NL¹L² sont en position péri, R¹ et L¹ peuvent représenter ensemble en outre un isopropylidène,
dans des huiles minérales, caractérisé en ce que l'on extrait la naphtylamine de formule I par traitement de l'huile minérale avec un milieu aqueux, et qu'on la couple dans la phase aqueuse, éventuellement en présence d'un tampon, avec un sel de diazonium dérivant d'une amine de la série des aminoantraquinones, pour former un colorant azoïque.

2. Procédé selon la revendication 1, caractérisé en ce que
R¹ et R² représentent chacun indépendamment l'un de l'autre, un atome d'hydrogène, un groupement alkyle en C₁-C₁₅, éventuellement substitué et pouvant être interrompu par 1 à 3 atomes d'oxygène en fonction éther, un groupement cyclohexyle, un groupement phényle éventuellement substitué ou bien R¹ et R² représentent ensemble avec l'atome d'azote qui les relie, un reste hétérocyclique saturé à 5 ou 6 maillons qui peut présenter un autre hétéroatome, et
deux des restes R³, R⁴, R⁵ et R⁶ représentent chacun un atome d'hydrogène et les autres représentent chacun indépendamment les uns des autres, un atome d'hydrogène, un groupement alkyle en C₁-C₄, alcoxy en C₁-C₄, hydroxy, nitro, alcanoylamino en C₁-C₄, benzoylamino, un reste de formule NL¹L², où L¹ et L² sont mis indépendamment l'un de l'autre pour un atome d'hydrogène ou un groupement alkyle en C₁-C₄, cyano ou carboxyle, où lorsque les restes NR¹R² et NL¹L² sont en position péri, R¹ et L¹ peuvent représenter ensemble en outre un isopropylidène.

3. Procédé selon la revendication 1, caractérisé en ce que R¹ représente un atome d'hydrogène, R² représente un groupement alkyle en C₁-C₁₅, pouvant être interrompu par 1 à 3 atomes d'oxygène en fonction éther, ou un groupement cyclohexyle et l'un des restes R³, R⁴, R⁵ et R⁶ représentent un groupement hydroxy ou alcoxy en C₁-C₄ et les autres représentent des atomes d'hydrogène.

4. Procédé selon la revendication 1, caractérisé en ce que l'on extrait la naphtylamine de formule I par traitement de l'huile minérale avec une solution aqueuse d'un sel de diazonium dérivant d'une amine de la série des aminoantraquinones, et qu'on la couple ensuite en présence d'un tampon, avec le sel de diazonium pour former un colorant azoïque.

5. Utilisation de naphtylamines de formule la 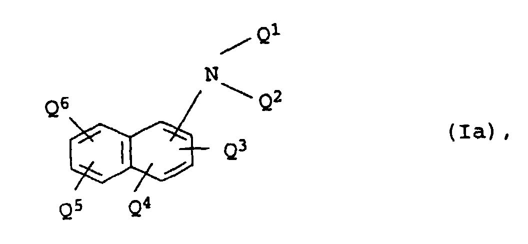 dans laquelle
Q¹ représente un atome d'hydrogène, Q² représente un groupement alkyle en C₁-C₁₅, pouvant être interrompu par 1 à 3 atomes d'oxygène en fonction éther, ou un groupement cyclohexyle et l'un des restes Q³, Q⁴, Q⁵ et Q⁶ représentent un groupement hydroxy ou alcoxy en C₁-C₄ et les autres représentent des atomes d'hydrogène, pour le marquage d'huiles minérales.

6. Huile minérale contenant une ou plusieurs des naphtylamines de formule la selon la revendication 5.
